# FASCICULE DE BREVET EUROPEEN

(11) **EP 0 456 708 B1**
(45) Date de publication et mention de la délivrance du brevet: **21.04.1993**
(21) Numéro de dépôt: 90902793.0
(22) Date de dépôt: 30.01.1990
(51) Int. Cl.: C12M 1/34, G01N 21/53, G01N 21/85

(54) **APPAREIL DE MESURE DE DENSITE OPTIQUE IN SITU**
VORRICHTUNG, UM DIE OPTISCHE DICHTE IN SITU ZU MESSEN
DEVICE FOR MEASURING OPTICAL DENSITY IN SITU

(30) Priorité: 31.01.1989 FR 8901165
(43) Date de publication de la demande: 21.11.1991
(73) Titulaire: SOCIETE NATIONALE ELF AQUITAINE, 92400 Courbevoie (FR)
(72) Inventeur: SAGNER, Max, F-75014 Paris (FR)
(74) Mandataire: Boillot, Marc
(86) Numéro de dépôt international: FR9000072
(87) Numéro de publication internationale: WO9008818

(56) Documents cités:
- DE-A- 2 412 038
- DE-A- 2 534 763
- Patent Abstracts of Japan, volume 10, no. 229 (P-485) (2285), 8 août1986
- Patent abstracts of Japan volume 12, no. 288 (P-741) (3135), 8 août 1988
- Patent Abstracts of Japan volume 9, no. 167 (P-372 (1980), 12 juillet 1985

## Description

La présente invention concerne un appareil pour mesurer la densité optique in situ d'un milieu réactionnel, en particulier dans un fermenteur.

La mesure de la biomasse est très importante pour le suivi et la conduite des bioréacteurs, ceci aussi bien dans les laboratoires de développement de procédé que dans les ateliers de production.

De façon usuelle, cette mesure s'effectue soit par prélèvement d'un échantillon du milieu réactionnel puis comptage au laboratoire, sous microscope, du nombre de cellules, ce qui est très long à savoir plus de 12 heures, soit encore par calcul de la densité optique qui est le logarithme du rapport entre l'intensité "entrante" d'un flux lumineux à son émission et l'intensité "sortante" de ce même flux après qu'il ait traversé sur une certaine longueur un échantillon du milieu réactionnel contenu dans le fermenteur. Pour une réaction donnée, la densité optique en transmission est une grandeur bien corrélée à la quantité de biomasse du milieu réactionnel et sa mesure permet d'évaluer indirectement la concentration bactérienne dudit milieu.

Dans un dispositif de mesure de densité optique fabriqué et commercialisé par la société suisse CHEMAP, la mesure s'effectue dans une cellule située à l'extérieur du fermenteur. Ce dispositif de mesure est relié au fermenteur par un fin tuyau de plusieurs dizaines de centimètres de long environ, par lequel est aspiré l'échantillon du milieu réactionnel sur lequel la mesure s'effectue, ledit tuyau restant en permanence ouvert sur le fermenteur. Le principe de fonctionnement de la cellule est assez similaire à celui d'une seringue, la cellule de mesure s'apparentant au réservoir de la seringue : on aspire d'abord une partie du liquide contenu dans le fermenteur jusqu'à la cellule, et, pour éviter que les bulles contenues dans ce liquide faussent la mesure de densité optique, on laisse reposer le liquide quelques dizaines de secondes, le temps que les bulles migrent à la partie supérieure de la cellule. On effectue ensuite la mesure en comparant l'intensité du flux lumineux à l'entrée de la cellule avec l'intensité de ce flux à la sortie de la cellule, puis, une fois la mesure effectuée, on réinjecte le liquide dans le fermenteur. Pour prendre en compte d'éventuelles variations de l'intensité de la source lumineuse émettant le flux lumineux, on mesure en même temps la densité optique à travers l'échantillon de liquide et l'intensité lumineuse émise par la source. On peut ainsi calibrer le résultat. De plus, un système de petits balais permet, sur ce dispositif, de nettoyer par raclage les surfaces optiques entre deux mesures.

Un premier inconvénient de cet appareil est son encombrement ; un deuxième inconvénient est qu'il existe un risque de défaut de stérilisation et, à cause du tuyau ouvert sur le milieu, de pollution de la fermentation. L'utilisation routinière de cet appareil peut poser des problèmes en production.

Dans le brevet US 4.725.148, on décrit un dispositif permettant la mesure in situ, donc sans prélèvements, de la densité optique du milieu réactionnel contenu dans un fermenteur. Le dispositif est ici à l'intérieur d'un système mécanique enclavé dans la paroi du fermenteur, de sorte que sa partie inférieure trempe dans le milieu réactionnel.

Dans la partie inférieure de l'appareil est ménagé en guise de cellule un espace ouvert sur le milieu réactionnel de sorte que celui-ci y circule librement. La mesure de biomasse s'effectue ici également par comparaison de l'intensité d'un flux lumineux émis et de l'intensité de ce même flux reçu après traversée sur une épaisseur connue du milieu réactionnel à étudier. La source lumineuse de ce dispositif est une diode laser semi-conductrice fournissant une intensité constante, de sorte qu'un calibrage n'est pas nécessaire, et le récepteur est une photodiode semi-conductrice. Un circuit de refroidissement limite la température du dispositif de sorte que l'appareil peut rester en place durant les phases de stérilisation. Pour éviter que les bulles viennent perturber la mesure, une grille est placée à l'entrée de la cellule. Cette grille ne doit pas être trop fine pour ne pas empêcher les grosses particules contenues dans le milieu réactionnel d'entrer dans la cellule : la mesure effectuée ne serait alors pas représentative de l'état réel dudit milieu, surtout en fin de fermentation. Les petites bulles, de diamètre inférieur au pas de la grille pourront donc venir perturber la mesure : si par exemple le taux d'agitation du milieu est modifié, le nombre et la grosseur des bulles varient et la mesure risque d'être différente. Un autre problème est que cette grille s'encrasse assez vite et qu'il n'est pas prévu de moyens pour son nettoyage.

Le dispositif de mesure de densité optique selon l'invention, tout en conservant les avantages de l'art antérieur permet de remédier à certains de leurs inconvénients.

Le principe utilisé consiste à enfermer un prélèvement du milieu réactionnel dans une cellule de mesure in situ, à attendre après fermeture de la cellule que les bulles se rassemblent dans la partie supérieure de la cellule, puis à effectuer la mesure de densité optique, par transmission, à la partie inférieure de celui-ci.

Le dispositif selon l'invention est constitué à cet effet d'une pièce principale formant le corps du dispositif dans lequel est ménagé un logement, d'une pièce mobile pouvant se loger dans le logement de la pièce principale et dans laquelle est ménagé une cavité ouverte sur l'extérieur de la pièce mobile par au moins deux orifices opposés pouvant être jointifs, te moyens optiques inclus dans la pièce principale, de part et d'autre du logement, pour conduire un flux lumineux d'une source à un analyseur de lumière, de moyens pour déplacer la pièce mobile d'une position ouverte pour laquelle le milieu à étudier circule librement dans la cavité de la pièce mobile à une position fermée pour laquelle la pièce mobile est enclavée dans le logement de la pièce principale de sorte que la cavité et son contenu, constituant l'échantillon du milieu à analyser sur lequel la mesure va s'effectuer, sont isolés du milieu à étudier, les moyens optiques étant adaptés en fonction de la place des orifices de la pièce mobile pour que le flux lumineux traverse le contenu de la cavité quand la pièce mobile est en position fermée. Des moyens pour étalonner les mesures et des moyens pour nettoyer les surfaces optiques à chaque mesure sont également prévus.

Les moyens d'étalonnage sont constitués d'au moins un barreau optique de référence, en verre ou en quartz par exemple, de caractéristiques -en particulier le coefficient de transmission- connues, et qui sera, momentanément au moins, placé sur le chemin optique du flux lumineux. Dans le cas où il y a plus d'un barreau d'étalonnage, ceux-ci seront préférentiellement de coefficients de transmission différents, ce qui permet un étalonnage sur plusieurs points et un balayage d'une plus grande étendue de dynamique qu'avec un barreau unique, et ce, sans démontage. Les barreaux d'étalonnage seront préférentiellement placés dans l'épaisseur de la pièce mobile.

Les moyens de nettoyage peuvent être constitués de racloirs en brosses, par exemple, placés sur la pièce mobile pour nettoyer les surfaces optiques de la pièce principale et de racloirs ou brosses situés sur la pièce principale pour nettoyer les surfaces optiques de la pièce mobile. Les moyens de nettoyage décrochent en particulier les petites bulles qui pourraient rester accrochées aux surfaces optiques.

Les éléments du dispositif selon l'invention sont résistants à des températures de plusieurs centaines de degrés pour que la stérilisation en laissant le dispositif en place soit possible.

Dans une forme de réalisation préférentielle, le prélèvement s'effectue par rotation de la pièce mobile autour d'un axe de rotation commandé par moteur.

Les moyens optiques peuvent être constitués d'un faisceau unique de fibres optiques bifurquées reliant le logement d'une part, à la source et à l'analyseur d'autre part, et d'un réflecteur situé à la surface du logement, en vis-à-vis de l'extrémité du faisceau de fibres optiques débouchant sur le logement, l'extrémité du faisceau de fibres et le réflecteur étant placés de façon à être en vis-à-vis chacun d'un orifice de la cavité de la pièce mobile quand celle-ci est en position fermée, les deux orifices étant opposés.

Selon une variante, le faisceau de fibres est constitué de fibres simples ayant toutes leur extrémité débouchant sur le logement de la pièce principale, la moitié d'entre elles, les fibres "aller", reliant le logement à la source, l'autre moitié, les fibres "retour" reliant le logement à l'analyseur de lumière, les fibres "aller" et les fibres "retour" étant réparties aléatoirement dans le faisceau.

Préférentiellement, la pièce mobile est un disque de bases parallèles, s'inscrivant dans un cercle dont l'épaisseur est égale à la largeur d'une entaille réalisée à l'extrémité de la pièce principale, perpendiculaire à l'axe de la pièce principale et constituant le logement.

La cavité peut présenter une symétrie de révolution d'axe perpendiculaire aux deux bases du disque constituant la pièce mobile et est ouverte sur l'extérieur par deux orifices de section circulaire : la cavité peut être, entre autres, un cylindre, un cône ou un double cylindre de section droite en forme de T.

La cavité ménagée dans la pièce mobile peut encore être un secteur ouvert sur l'extérieur par la base supérieure, la base inférieure et la face latérale de la pièce mobile, les deux orifices de la cavité de la pièce mobile étant jointifs, et les bases de la pièce mobile prenant la forme d'un V ou d'un U.

Le dispositif est de très petite dimension, de l'ordre de 80 mm de longueur et de 25 mm de diamètre. Il peut être placé soit dans un des bossages normalement aménagé à la base des fermenteurs et qui sont destinés à recevoir un ou plusieurs appareils de mesure, soit comme le dispositif décrit dans le brevet US 4.725.148, à l'intérieur d'un système mécanique enclavé dans la paroi du fermenteur. Le dispositif qui peut, par exemple, être monté sur un couvercle étanche qui se visse au bossage ou sur le système mécanique, peut ainsi aisément s'enlever, pour le nettoyage entre autres. Une fois mis en place, le dispositif trempe dans le milieu réactionnel contenu dans le fermenteur, surtout sa base inférieure où se trouve la pièce mobile.

Le dispositif selon l'invention permet donc le suivi in situ et en temps réel, en particulier de la fermentation, tout en s'affranchissant des problèmes de pollution de la mesure du fait de la présence de micro-bulles et de résidus de fermentation, du parasitage par la lumière ambiante qui crée dans les systèmes ouverts un bruit de mesure que l'on est obligé d'éliminer par modulation, opération qui n'est pas nécessaire ici, ou de variation d'intensité lumineuse. Grâce à sa petite taille et la facilité pour le nettoyer et le stériliser, ce dispositif ne pose pas de problème en production.

D'autres caractéristiques et avantages de la présente invention apparaîtront plus clairement à la lecture de la description ci-après faite en référence aux figures annexées dans lesquelles :
- la figure 1 est une coupe longitudinale du dispositif selon une réalisation préférentielle, permettant de comprendre comment le dispositif est placé au travers de la paroi de la cuve contenant le milieu à étudier,
- les figures 2A et 2B représentent deux coupes transversales au niveau de la pièce mobile, pour deux formes de réalisation possible de la pièce mobile animée d'un mouvement de rotation, l'une avec base circulaire et cavité cylindrique (fig. 2A), l'autre avec base triangulaire et cavité en forme de secteur (fig. 2B),
- les figures 3A et 3B représentent deux coupes longitudinales de l'extrémité du dispositif au niveau de la pièce mobile pour deux autres formes de réalisation possible de ladite pièce mobile et de la cavité.

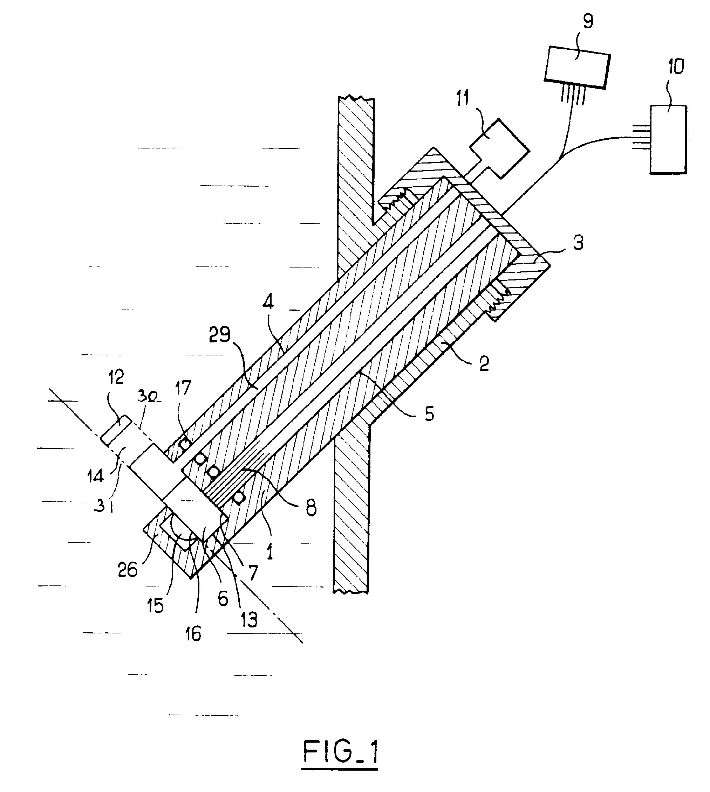

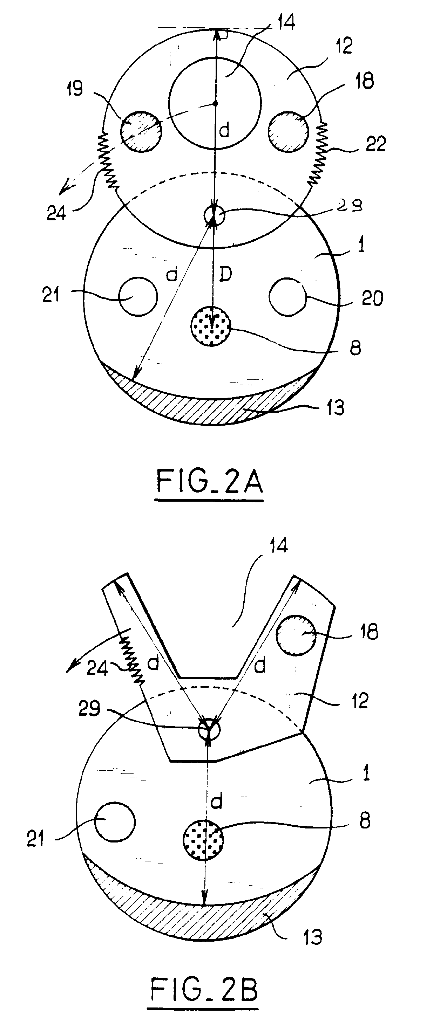

Un dispositif selon l'invention, tel qu'il peut se présenter une fois mis en place, est représenté en coupe longitudinale sur la figure 1 et en coupe transversale sur la figure 2A. Le dispositif comporte une pièce principale 1 notamment en métal inoxydable, qui est un cylindre d'environ 80 mm de longueur dont la section circulaire a un diamètre de 25 mm par exemple. Cette pièce 1 est maintenue à un bossage 2 de la paroi de la cuve du fermenteur par un couvercle 3 dont la pièce 1 est solidaire et qui est vissé au bossage 2. Sur un diamètre quelconque de la section droite de la pièce principale -diamètre définissant le plan de coupe de la figure 1- deux trous de même longueur 4 et 5 sont percés dans la pièce principale, de part et d'autre du centre du diamètre, à des distances quelconques.

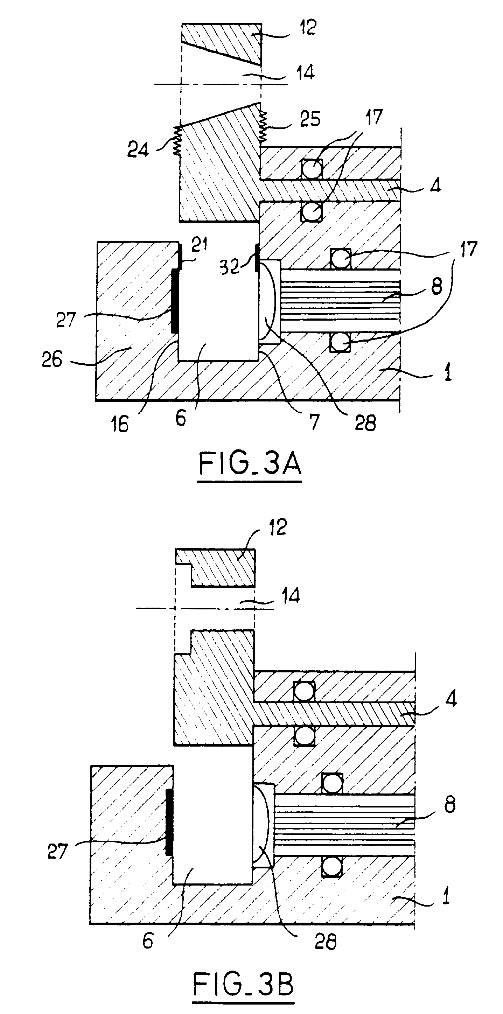

Les trous, de même direction que l'axe de la pièce 1, traversent la pièce principale sur presque toute sa longueur, la partie non trouée, opposée à l'extrémité rattachée au couvercle 3 étant définie comme la partie inférieure de la pièce. A une dizaine de millimètres de l'extrémité inférieure de la pièce principale, une profonde entaille 6 suivant un plan perpendiculaire à l'axe de la pièce principale est réalisée. Cette entaille a pour axe de symétrie un axe parallèle au diamètre passant par les trous 4 et 5, et a pour largeur entre 5 et 15 mm. Elle constitue le logement de la pièce principale où va se loger la pièce mobile. L'entaille est limitée par deux flancs parallèles, le flanc supérieur 7 et le flanc inférieur 16. L'entaille sépare la partie supérieure de la pièce principale, la plus proche de la paroi de la cuve, de sa partie inférieure 26. Les surfaces deux deux flancs 7 et 16 sont en principe égales, toutefois la surface du flanc 16 peut se trouver diminuée du fait de l'élimination du volume non utile de la partie inférieure 26, ce pour économiser de la matière, comme on le montre sur les figures 1, 3A et 3B.

Le fond de l'entaille est usinée suivant un arc de cercle dont le centre est situé sur l'extrémité inférieure de l'axe de rotation 29, à son attache à la pièce mobile, et dont le rayon est supérieur à celui de la section droite de la pièce principale. Ce rayon est tel que l'épaisseur du fond 13 est telle que ce fond offre une résistance suffisante fonction, entre autres, de la force exercée sur la partie inférieure de la pièce principale. Le fond 13 liant la partie supérieure de la pièce principale à sa partie inférieure et dont le fond de l'entaille constitue une face a une section en forme de croissant de lune, comme on l'a représenté sur les figures 2A et 2B.

Chacun des trous 4 et 5 débouche sur le flanc 7 supérieur de l'entaille. Le trou 5, qui est celui qui débouche au plus profond de l'entaille, contient un faisceau 8 de fibres optiques. Au débouché sur l'entaille, les fibres sont groupées collées et polies, leur surface coïncidant exactement avec le flanc de l'entaille. A l'extrémité supérieure du trou 5, la moitié des fibres sont reliées, au travers du couvercle 3, à une source lumineuse 9, la seconde moitié des fibres étant reliées, au travers du couvercle 3, à un photomètre 10, les fibres reliées à la source ou à l'analyseur étant reparties aléatoirement dans le faisceau 8.

Comme on l'a représenté sur la figure 1, le faisceau de fibres simples peut être remplacé par un faisceau de fibres bifurquées présentant trois branches reliées en un point de couplage. L'une est empruntée à l'aller et au retour par le flux lumineux et est située dans le trou 5, la seconde relie le point de couplage à la source lumineuse 9, la troisième relie le point de couplage au photomètre 10. Le couplage des branches s'effectue au niveau du couvercle 3 ou à l'extérieur du bossage. Les source et photomètre sont extérieurs à la cuve.

Les fibres peuvent être réalisées en quartz, résistant à des températures de plusieurs centaines de degrés.

Dans le second trou 4, vient se loger un axe de rotation 29 relié à travers le couvercle de fixation 3 à un moteur de commande 11 extérieur à la cuve, et à son autre extrémité à un point décentré d'une pièce mobile 12 cylindrique, notamment en métal inoxydable, en butée contre le flanc supérieur 7 de l'entaille. Cette pièce mobile est un disque dont l'épaisseur est égale à la largeur de l'entaille 6.

Le diamètre de la pièce mobile est supérieur à la distance D qui existe entre l'axe de rotation et le faisceau optique, et est inférieur au diamètre de la pièce principale.

La distance d entre l'axe de rotation 29 et le point le plus éloigné du disque 12 sera égale ou inférieure au rayon du cercle selon lequel le fond de l'entaille est usiné, de sorte que la pièce mobile puisse effectuer une rotation de 180° autour de l'axe de rotation. Sur les exemples de réalisation décrits ici, les dimensions des pièces sont calculées de façon que la face latérale de la pièce mobile et le fond de l'entaille soient jointifs pour une position du disque, dite "fermée", obtenue après rotation de 180° par rapport à la position, dite "ouverte" représentée sur les figures 2A ou 2B.

La pièce mobile est percée sur toute son épaisseur d'un trou 14 cylindrique de diamètre supérieur à celui du faisceau de fibres optiques, de manière à définir une cavité ouverte sur l'extérieur de la pièce mobile par deux orifices 30 et 31 circulaires de même surface, situés chacun sur une des faces du disque et opposés. Ainsi, le fluide dans lequel trempe la partie inférieure de l'appareil, y compris la pièce mobile, peut circuler librement dans la cavité, pour certaines positions du disque, dites positions ouvertes.

La distance entre le centre de cette cavité et l'axe de rotation est égale à la distance D qui existe entre l'axe de rotation et le faisceau de fibres optiques de sorte qu'il existe une position particulière, au cours de la rotation du disque autour de l'axe de rotation, dite position fermée, pour laquelle le cylindre 14 est exactement dans le prolongement du faisceau de fibres optiques.

Un miroir convexe réfléchissant à surface extérieure plane 15 est incrusté dans le flanc inférieur de l'entaille, exactement dans le prolongement du faisceau de fibres 8. Ainsi en position fermée du dispositif, le réflecteur, la cavité cylindrique et ses orifices et l'extrémité du faisceau sont alignés, et le flux lumineux, amené par les fibres "aller" du faisceau 8, vient se réfléchir sur ce miroir 15 après avoir traversé la cavité 14 ménagée dans la pièce mobile contenant un échantillon du milieu à étudier, et est renvoyé sur l'extrémité du faisceau pour être reconduit par les fibres "retour" vers l'analyseur de lumière 10, ici un photomètre.

L'étanchéité au niveau de l'axe de rotation et du faisceau de fibres optiques est assurée par des joints 17 toriques placés autour de l'axe de rotation 29 et du faisceau 8 à proximité du flanc 7 supérieur de l'entaille.

Soit D la distance entre l'axe de rotation et le centre du trou contenant le faisceau de fibres optiques. A la distance D de l'axe de rotation, deux barreaux de verre 18 et 19 cylindriques, de longueur égale à l'épaisseur de la pièce mobile, et de diamètre sensiblement égal au diamètre du faisceau de fibres optiques, sont inclus dans la pièce mobile, suivant des axes perpendiculaires aux bases de la pièce.

Ainsi, en coupe transversale, telle celle représentée sur la figure 2A, les barreaux et l'extrémité du faisceau optique sont situés sur un même cercle de rayon D et de centre l'axe de rotation, de sorte que lors de la rotation de la pièce mobile autour de l'axe de rotation, chacun des barreaux se trouve à un moment dans le prolongement du faisceau de fibres optiques, entre l'extrémité du faisceau et le miroir 15. Les barreaux sont en quartz, traités de façon à ce que leurs coefficients de transmission soient différents, pour balayer une plus grande étendue de dynamique qu'avec un barreau unique, et ce sans démontage.

Sur ce même cercle de centre l'axe de rotation et de rayon D sont placés sur chacun des flancs de l'entaille de la pièce principale deux petites brosses en poils 20 et 21, 32 et 33. Les brosses sont destinées à nettoyer les surfaces des barreaux optiques lors de la rotation de la pièce mobile.

Des petits balais 22, 23, 24, 25 destinés à nettoyer les surface du faisceau de fibres optiques et surface du miroir sont fixés sur la paroi latérale de la pièce mobile deux à deux et dos à dos de façon que les poils dépassent légèrement des bases supérieures et inférieures de la pièce mobile

Les balais sont placés sur le pourtour de la pièce mobile de telle sorte qu'ils passent sur les surfaces optiques -extrémité du faisceau optique et surface du réflecteur- lors de la rotation de la pièce mobile.

Les figures 2B, 3A et 3B montrent d'autres modes de réalisation possibles de la pièce mobile animée d'un mouvement de rotation, en particulier différentes formes que peut prendre la cavité qui contiendra l'échantillon de liquide sur lequel la mesure s'effectuera.

Sur la figure 2B par exemple, la pièce mobile a grossièrement la forme d'un U aux branches épaisses, le volume est ouvert sur le milieu réactionnel par les bases supérieures et inférieures de la pièce et également par le côté. Un seul barreau d'étalonnage est prévu ici. Dans ce cas, le dispositif sera avantageusement incliné ou orienté de façon que ces bulles se rassemblent dans un angle, dit supérieur, du secteur, la mesure de densité s'effectuant à la partie inférieure de celui-ci. Sur la figure 3A, le volume 14 est un cône divergent d'axe de symétrie de même direction que l'axe de la pièce principale. Sur la figure 3B, le volume présente une section en forme de T. Ces deux dernières géométries présentent l'avantage que, indépendamment de la position du système, les bulles sont piégées dans la partie haute du dièdre ou dans celle de plus grand diamètre. Il n'est donc plus nécessaire de positionner spécialement le système lors de la fixation sur le fermenteur.

Dans ces deux derniers cas, on peut remplacer avantageusement le miroir convexe de la figure 1 par un miroir plan 27, et intercaler entre l'extrémité du faisceau de fibre une lentille collimatrice 28 résistant aux températures élevées de l'ordre de 750°F.

Bien entendu, la présente invention n'est pas limitée aux modes de réalisation décrits. Elle s'étend au contraire à toute variante incluse dans le cadre des revendications ci-après.

En particulier, on peut envisager un dispositif où le déplacement de la pièce mobile ne serait plus une rotation mais le simple glissement d'une pièce par exemple cubique, vide en partie, dans une pièce principale dans laquelle on aurait ménagé un logement destiné à recevoir la pièce mobile cubique.

## Revendications

1. Dispositif pour la mesure in situ de la densité optique d'un milieu, caractérisé en ce qu'il est constitué
- d'une pièce principale (1) formant le corps du dispositif, dans lequel est ménagé un logement (6),
- d'une pièce mobile (12) pouvant se loger dans ledit logement et dans laquelle est ménagée une cavité (14) ouverte sur l'extérieur de la pièce mobile par au moins deux orifices (30, 31) opposés pouvant être jointifs,
- de moyens optiques (8, 15), (8, 28, 27) inclus dans la pièce principale, de part et d'autres du logement, pour conduire un flux lumineux d'une source à un analyseur de lumière,
- de moyens pour déplacer la pièce mobile d'une position ouverte pour laquelle le milieu à étudier circule librement dans la cavité de la pièce mobile, à une position fermée pour laquelle la pièce mobile est enclavée dans le logement de la pièce principale de sorte que la cavité et son contenu, constituant l'échantillon du milieu à analyser sur lequel la mesure va s'effectuer, sont isolés du milieu à étudier, les moyens optiques étant adaptés en fonction de la place des orifices de la pièce mobile pour que le flux lumineux traverse le contenu de la cavité quand la pièce mobile est en position fermée.

2. Dispositif selon la revendication 1 caractérisé en ce qu'il comporte des moyens d'étalonnage (18, 19) de l'intensité du flux lumineux et des moyens de nettoyage (20, 21, 22, 23, 24, 25, 32, 33) des surfaces optiques.

3. Dispositif selon la revendication 2 caractérisé en ce que les moyens d'étalonnage sont constitués d'au moins un barreau optique de caractéristiques connues placé momentanément au moins sur le chemin optique du flux lumineux.

4. Dispositif selon la revendication 3 caractérisé en ce que les moyens d'étalonnage consistent en plusieurs barreaux d'étalonnage présentant des coefficients de transmission différents.

5. Dispositif selon les revendication 3 ou 4 caractérisé en ce que les barreaux d'étalonnage sont placés dans l'épaisseur de la pièce mobile.

6. Dispositif selon la revendication 2 caractérisé en ce que les moyens de nettoyage sont constitués de racloirs ou brosses placés sur la pièce mobile pour nettoyer les surfaces optiques de la pièce principale, et de racloirs ou brosses placés sur la pièce principale pour nettoyer les surfaces optiques de la pièce mobile.

7. Dispositif selon la revendication 1 ou 2 caractérisé en ce qu'il est constitué d'éléments résistants à la température de stérilisation.

8. Dispositif selon la revendication 1 ou 2 caractérisé en ce que les moyens de déplacement de la pièce mobile comportent un axe de rotation (29), lié à la pièce mobile et un moteur (11) assurant la rotation dudit axe.

9. Dispositif selon la revendication 1 ou 2 caractérisé en ce que les moyens optiques sont constitués d'un faisceau de fibres (8) bifurquées reliant le logement (6) d'une part à la source (9) et l'analyseur de lumière (10) d'autre part, et d'un réflecteur (15, 27) situé à la surface du logement en vis-à-vis de l'extrémité du faisceau de fibres.

10. Dispositif selon la revendication 1 caractérisé en ce que la pièce mobile est un disque dont les bases s'inscrivent dans un cercle et dont l'épaisseur est égale à la largeur d'une entaille réalisée pris d'une extrémité de la pièce principale, perpendiculairement à l'axe longitudinal de la pièce principale, et constituant le logement.

11. Dispositif selon la revendication 10 caractérisé en ce que la cavité de la pièce mobile présente un axe de symétrie perpendiculaire aux bases du disque, laquelle cavité est ouverte sur l'extérieur par deux orifices de section circulaire.

12. Dispositif selon la revendication 10, caractérisé en ce que la cavité ménagée dans la pièce mobile est un secteur de ladite pièce.

## Claims

1. Device for measuring in situ the optical density of a medium, characterised in that it consists of:
- a main part (1) forming the body of the device in which a housing (6) is provided;
- a movable part (12) which can be accommodated in the said housing and in which there is provided a cavity (14) open on the exterior of the movable part by means of at least two apertures (30, 31) which are opposite and can be contiguous;
- optical means (8, 15), (8, 28, 27) included in the main part, on either side of the housing, in order to conduct a luminous flux from a light source to a light analyzer;
- means for displacing the movable part from an open position, for which the medium to be studied circulates freely in the cavity in the movable, part, to a closed position, for which the movable part is enclosed in the housing of the main part such that the cavity and the contents thereof, forming the sample of the medium to be analyzed on which measurements will be taken, are isolated from the medium to be studied, the optical means being adapted as a function of the location of the apertures in the movable part such that the luminous flux passes through the contents of the cavity when the movable part is in the closed position.

2. Device according to Claim 1, characterised in that it comprises means (18, 19) for calibrating the intensity of the luminous flux and means (20, 21, 22, 23, 24, 25, 32, 33) for cleaning the optical surfaces.

3. Device according to Claim 2, characterised in that the calibrating means consist of at least one optical bar having known characteristics and placed temporarily at least in the optical path of the luminous flux.

4. Device according to Claim 3, characterised in that the calibrating means consist of a plurality of calibrating bars having different coefficients of transmission.

5. Device according to either of Claims 3 or 4, characterised in that the calibrating bars are placed in the thickness of the movable part.

6. Device according to Claim 2, characterised in that the cleaning means consist of scrapers or brushes placed on the movable part so as to clean the optical surfaces of the main part, and of scrapers or brushes placed on the main part so as to clean the optical surfaces of the movable part.

7. Device according to either of Claim 1 or 2, characterised in that it consists of components which are resistant to the sterilisation temperature.

8. Device according to either of Claims 1 or 2, characterised in that the means for displacing the movable part comprise a rotary shaft (29) connected to the movable part and a motor (11) rotating the said shaft.

9. Device according to either of Claims 1 or 2, characterised in that the optical means consist of a bundle (8) of bifurcated fibres connecting the housing (6) on the one hand to the light source (9) and analyzer (10) on the other hand, and a reflector (15, 27) located on the surface of the housing opposite the end of the fibre bundle.

10. Device according to Claim 1, characterised in that the movable part is a disc of which the bases form a circle and of which the thickness is equal to the width of a notch provided adjacent one end of the main part, perpendicularly to the longitudinal axis of the main part, and constituting the housing.

11. Device according to Claim 10, characterised in that the cavity in the movable part has an axis of symmetry which is perpendicular to the bases of the disc, the cavity being open on the exterior by means of two apertures having a circular cross-section.

12. Device according to Claim 10, characterised in that the cavity provided in the movable part is a sector of the said part.

## Patentansprüche

1. Vorrichtung zur In-Situ-Messung der optischen Dichte eines Mediums, gekennzeichnet durch
- ein den Körper der Vorrichtung bildendes Hauptglied (1), in dem eine Aufnahme (6) ausgespart ist,
- einem beweglichen Glied (12), das in der Aufnahme aufgenommen werden kann und in dem ein Hohlraum (14) ausgespart ist, der zur Außenseite des beweglichen Gliedes über wenigstens zwei gegenüberliegende Öffnungen (30, 31) offen ist, die aneinanderstoßen können,
- optischen Einrichtungen (8, 15), (8, 28, 27), die in dem Hauptglied auf beiden Seiten der Aufnahme enthalten sind, um einen Lichtstrom von einer Quelle zu einem Lichtanalysator zu leiten, und
- Einrichtungen zur Verschiebung des beweglichen Gliedes aus einer offenen Stellung, in der das zu untersuchende Medium frei in dem Hohlraum des beweglichen Gliedes zirkuliert, in eine geschlossene Stellung, in der das bewegliche Glied in der Aufnahme des Hauptgliedes so eingeschlossen ist, daß der Hohlraum und sein Inhalt, der aus der Probe des zu analysierenden Mediums besteht, mit dem die Messung durchgefährt wird, von dem zu untersuchenden Medium isoliert sind, wobei die optischen Einrichtungen entsprechend der Lage der Öffnungen des beweglichen Gliedes angepaßt sind, damit der Lichtstrom den Inhalt des Hohlraums durchquert, wenn das bewegliche Teil sich in der geschlossenen Stellung befindet.

2. Vorrichtung nach Anspruch 1, dadurch gekennzeichnet, daß sie Einrichtungen (18, 19) zur Eichung der Intensität des Lichtstroms und Einrichtungen (20, 21, 22, 23, 24, 25, 32, 33) zur Reinigung der optischen Flächen aufweist.

3. Vorrichtung nach Anspruch 2, dadurch gekennzeichnet, daß die Einrichtungen zur Eichung von wenigstens einem optischen Gitter mit bekannten Eigenschaften gebildet werden, das vorübergehend wenigstens auf dem optischen Weg des Lichtstroms angeordnet ist.

4. Vorrichtung nach Anspruch 3, dadurch gekennzeichnet, daß die Einrichtungen zur Eichung von mehreren Eichgittern gebildet werden, die unterschiedliche Durchlässigkeitsfaktoren aufweisen.

5. Vorrichtung nach Anspruch 3 oder 4, dadurch gekennzeichnet, daß die Eichgitter in der Dicke des beweglichen Gliedes angeordnet sind.

6. Vorrichtung nach Anspruch 2, dadurch gekennzeichnet, daß die Reinigungseinrichtungen von Abstreifern oder Bürsten, die auf dem beweglichen Glied zur Reinigung der optischen Flächen des Hauptgliedes angeordnet sind, und von Abstreifern oder Bürsten gebildet werden, die auf dem Hauptglied zur Reinigung der optischen Flächen des beweglichen Gliedes angeordnet sind.

7. Vorrichtung nach Anspruch 1 oder 2, dadurch gekennzeichnet, daß sie von Elementen gebildet wird, die gegen die Sterilisationstemperatur beständig sind.

8. Vorrichtung nach Anspruch 1 oder 2, dadurch gekennzeichnet, daß die Einrichtungen zur Verschiebung des beweglichen Gliedes eine mit dem beweglichen Glied verbundene Drehachse (29) und einen Motor (11) umfassen, der die Drehung der Achse sicherstellt.

9. Vorrichtung nach Anspruch 1 oder 2, dadurch gekennzeichnet, daß die optischen Einrichtungen von einem sich gabelnden Faserbündel (8), das die Aufnahme (6) einerseits mit der Quelle (9) und andererseits mit dem Lichtanalysator (10) verbindet, und von einem Reflektor (15, 27) gebildet werden, der an der Flache der Aufnahme angeordnet ist, die dem Ende des Faserbändels gegenüberliegt.

10. Vorrichtung nach Anspruch 1, dadurch gekennzeichnet, daß das bewegliche Glied eine Scheibe ist, deren Grundflächen in einen Kreis eingeschrieben sind, und deren Dicke gleich der Breite eines Schlitzes ist, der in der Nähe eines Endes des Hauptgliedes senkrecht zur Längsachse des Hauptgliedes vorgesehen ist und die Aufnahme bildet.

11. Vorrichtung nach Anspruch 10, dadurch gekennzeichnet, daß der Hohlraum des beweglichen Gliedes eine Symmetrieachse hat, die zu den Grundflächen der Scheibe senkrecht ist, wobei der Hohlraum zur Außenseite über zwei Öffnungen offen ist, die einen kreisförmigen Querschnitt haben.

12. Vorrichtung nach Anspruch 10, dadurch gekennzeichnet, daß der in dem beweglichen Glied ausgesparte Hohlraum ein Sektor des Gliedes ist.
